# EUROPEAN PATENT APPLICATION

(11) **EP 1 997 448 A1**
(43) Date of publication of application: **03.12.2008**
(21) Application number: 08009471.7
(22) Date of filing: 23.05.2008
(51) Int. Cl.: A61B 17/70, A61B 17/88

(54) **Medical device for positioning bone elements, in particular vertebrae, relative to each other, as well as a tool for the placement, part by part, of such a medical device**

(30) Priority: 31.05.2007 NL 1033910
(71) Applicant: Baat Holding B.V., 7553 LZ Hengelo (NL)
(72) Inventor: Bonnema, Thomas Allard Xander, 7557 BX Hengelo (NL); Ter Braak, Hubertus Paul Maria, 7482 MA St. Isidorushoeve (NL); Van der Kouwe, Danielle Elisabeth, 7557 RR Hengelo (NL); De Roose, Joseph, 9940 Evergem (BE); Uyttendaele, Dirk, 9230 Wetteren (BE); Poffyn, Bart, 9820 Bottelare (BE)
(74) Representative: Dorna, Peter

(57) **Abstract**

The invention relates to a medical device for positioning bone elements, in particular vertebrae, relative to each other, comprising at least one assembly built up of two or more anchoring elements, which can each be implanted in a bone element with a first end thereof, as well as an elongated connecting element, which can be connected to the other ends of the anchoring elements projecting from the bone element in question.

The object of the invention is to provide an improved medical device as referred to in the introduction, placement of which requires a simpler procedure and thus less drastic surgery, which is less burdening for the patient, therefore.

According to the invention, the medical device is characterised in that a tool is provided for use in the placement, part by part, of the medical device, which tool comprises at least two hollow, tubular guide members, which guide members can each be positioned into abutment with a bone element for guiding the anchoring elements through the guide member and inserting said anchoring elements in the bone element, as well as an adjusting mechanism, which can be connected to the other ends of the guide members for positioning the guide members and the bone elements relative to each other.

## Description

The invention relates to a medical device for positioning bone elements, in particular vertebrae, relative to each other, comprising at least one assembly built up of two or more anchoring elements, which can each be implanted in a bone element with a first end thereof, as well as an elongated connecting element, which can be connected to the other ends of the anchoring elements projecting from the bone element in question.

Such medical devices are generally known, they are used for the relative positioning or fixation of vertebrae positioned adjacent to each other, whose intervertebral disc is damaged and/or has lost its function. Examples of such medical solutions are shown in WO 01/45576 and WO 02/102259 or NL-1029568. The placement of such a medical device requires a surgical procedure with major consequences for the patient, for which the vertebrae under the patient's skin need to be exposed.

Moreover, in many cases one or more ribs are broken and removed - in order to obtain a certain degree of freedom of movement in the surgical area - before the medical device is connected, part by part, to the vertebrae in question. Not only does the surgical procedure take a great deal of time, but the recovery period may be long and the trauma to the patient may be considerable.

The object of the invention is to provide an improved medical device as referred to in the introduction, the placement of which requires a simpler procedure and thus less radical surgery, which is less burdening for the patient, therefore.

According to the invention, the medical device is characterised in that a tool is provided for use in the placement, part by part, of the medical device, which tool comprises at least two hollow, tubular guide members, which guide members can each be positioned into abutment with a bone element for guiding the anchoring elements through the guide member and inserting said anchoring elements in the bone element, as well as an adjusting mechanism, which can be connected to the other ends of the guide members for positioning the guide members and the bone elements relative to each other.

The surgical area can be significantly reduced by using a hollow, tubular guide member as a placement tool. The patient is only burdened with two, relatively small surgical wounds at the location of the vertebra(e) to be treated, as a result of which the extent of trauma and other inconvenience is reduced and recovery is accelerated.

According to one embodiment, the first end of each guide member is arranged for receiving a cramp-like element, which can be driven into the bone element. In this way a solid and secure anchoring of the tool during the placement of the medical device according to the invention is realised, thereby reducing the risk of incorrect placement of the device.

According to another embodiment, the ends of the anchoring elements projecting from the bone element are each provided with a screw thread, which is to mate with a nut to be guided through the guide member, which nut functions to lock a connecting ring likewise to be guided through the guide member, which can be fitted over the end of the anchoring elements that projects from the bone element. Since the elongated connecting element further comprises two element parts to be guided by each guide member and to be connected to a connecting ring, as well as a connecting element for interconnecting said element parts, a reliable connection between the two anchoring elements can be realised in an adequate and secure manner with this embodiment.

According to the invention, the reliable connection between the anchoring elements by means of the element parts can be realised in that, more specifically, the element parts and the connecting rings can be connected together by connecting means built up of hook-shaped members formed on the connecting rings or the element parts, which hook-shaped members hook round cams formed on the element parts or on the connecting rings.

The connection between the element parts can be guaranteed in a simple and reliable manner in that one element part is bar-shaped and the other element part is spatula-shaped. In this way an adequate retainment or locking of the bar-shaped element part in the spatula-shaped element part can be realised.

In another functional embodiment, in order to facilitate connecting the element parts together, each guide member is partially open in longitudinal direction near its first end for the purpose of releasing and interconnecting the element parts connected to the connecting ring in question.

The invention further relates to a tool for use in the placement, part by part, of a medical device as described above.

The invention will not be explained in more detail with reference to a drawing, in which:
Figure 1 shows an embodiment of a medical device according to the prior art;
Figure 2 shows an embodiment of the medical device according to the invention;
Figures 3a-3d are partial views of the tool according to the invention for use in the placement of a medical device according to the invention;
Figures 4a-4c are further partial views of a medical device according to the invention;
Figure 5 shows another embodiment of the medical device according to the invention.

For a better understanding of the invention, like parts will be indicated by identical numerals in the description below.

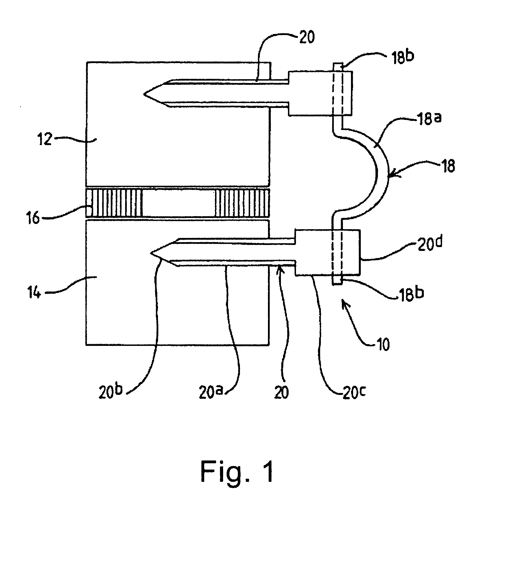

In Figure 1, numerals 12 and 14 schematically indicate two vertebrae, which are separated by an intervertebral disc 16. In the situation in which the intervertebral disc 16 is damaged or otherwise affected it may be desirable to position the vertebrae 12 and 14 located above and below, respectively, said intervertebral disc 16 relative to each other, to which end use is made in Figure 1 of a medical device built up at least of an assembly comprising two anchoring elements 20, which can be implanted in the vertebrae 12 and 14 with their distal ends 20a and 20b, respectively. A connecting element 18 may be provided between the two adjacent anchoring elements 20 at the other ends 20c-20d of the anchoring elements 20, which connecting element can be attached with its ends 18b to the two other ends 20c-20d of the anchoring elements 20 that extend from the bone elements.

Although the medical device is used with an affected intervertebral disc 16 in Figure 1, the medical device can also be used with an affected vertebra. In that situation the anchoring elements 20 will be implanted in the vertebrae located on either side of the affected vertebra.

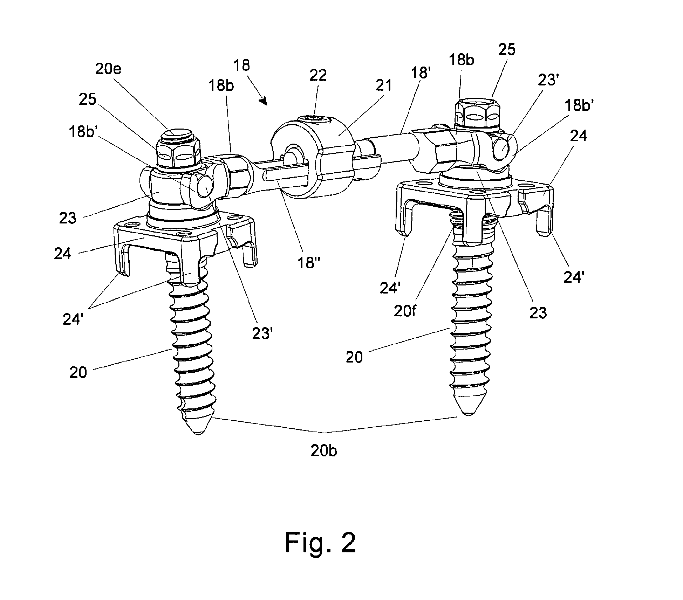

Figure 2 shows an embodiment of a medical device according to the invention, with reference to which it is noted that parts of the device corresponding to the parts shown in Figure 1 will be indicated by the same numerals. In the embodiment according to the invention as shown in Figure 2 the connecting element 18 is divided into two elements 18' and 18", which can be connected together by means of a clamping element 21 and a securing nut 22. The two screws 20 are implanted in different vertebrae.

Numeral 24 indicates a cramp element provided with projecting pins 24', whose function will be explained hereinafter.

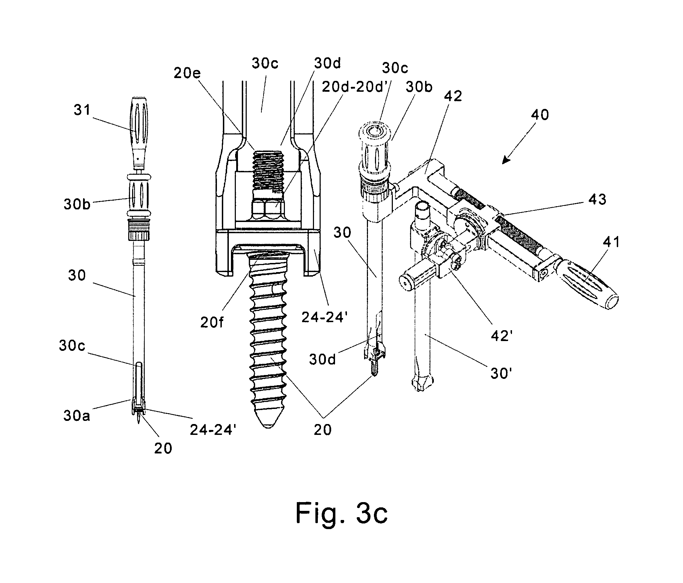

The various element parts 18'-18" of the connecting element 18 are connected to the end 20d of the anchoring element 20 that projects from the bone, using connecting means 18b'-23'. The part 20d of the anchoring elements 20 that projects from the bone element or vertebra is provided with an external screw thread 20e (see Figure 3c), onto which a nut 25 can be screwed, by means of which nut a connecting ring 23 previously placed over the end 20d is locked or retained. The connecting ring 23 is provided with projecting parts or cams 23', which function as part of connecting means that mate with a hook-shaped member 18b', which forms part of the end 18b of the element parts 18'-18".

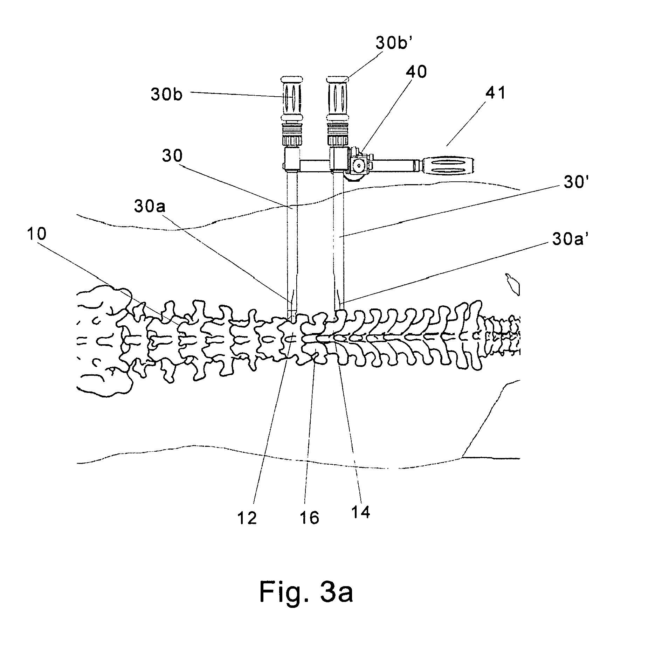

Figures 3A-3D show the steps of the method for placing or providing the medical device according to the invention, in which use is made of a specific tool, which is indicated at 30-30' in Figures 3a-3d (and further).

While in the prior art a large part of the back and the vertebral column for the ribs must be opened for surgically placing the medical device as shown in Figure 1, the object of the medical device according to the invention is to use less radical surgery for this purpose, so that the burden and extent of the inconvenience to the patient is significantly reduced.

According to the invention, use is made of a tool 30-30', which is configured as a hollow, tubular guide member which is to be placed into contact with the vertebra 12-14 in question through the skin. In this way the patient is only burdened with two minor surgical wounds, so that the extent of trauma is significantly reduced, thereby effecting a better or speedier recovery.

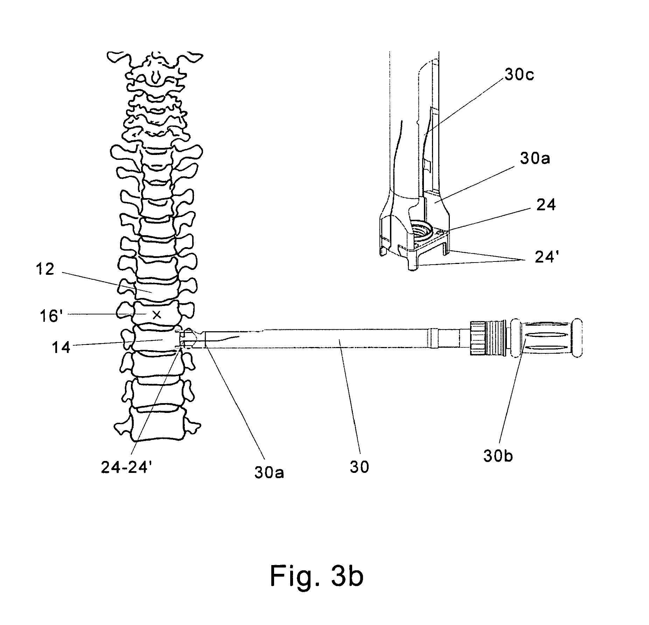

As shown in Figure 3B, the end 30a of the hollow, tubular guide member is arranged for receiving a cramp-like element 24 provided with teeth 24', which is to be inserted into the bone or vertebral column 14-12 by means of the guide member 30. The cramp-like element 24 functions as an anchoring element and orientation point for the guide member 30 for the placement, part by part, of the medical device according to the invention as shown in Figure 2.

The cramp-like element 24 has an opening provided with an internal screw thread (not shown in the Figures). The anchoring element 20 is provided with an external screw thread 20f (see Figures 2 and 3c), which mates with the internal screw thread of the cramp element 24 when the anchoring element is being screwed into the bone element 12-14. In this way a solid and reliable fixation of the anchoring element 20, the cramp elements 24 and the guide member 30-30' is guaranteed.

As shown in the Figures, the guide member 30 is provided with a hollow passage 30c, which functions to guide the various parts of the medical device according to the invention.

The various parts are inserted via the other end 30b of the guide member 30, which projects from the bone element, using an auxiliary tool 31 that forms part of the aforesaid tool, which auxiliary tool is bar-shaped and which can be guided through the hollow passage 30c of the guide member 30 in the direction of the first end 30a and the bone element 14-12.

As is clearly shown in Figure 3C, the auxiliary tool 31 functions to guide the anchoring element 20 through the hollow guide member 30, which anchoring element can be screwed into the bone or the vertebra 14-12 via an opening (not shown) formed in the cramp element 24. As a result, the part 20d of the anchoring elements 20 projecting from the bone element is provided with a faceted portion 20d', which mates with a corresponding, complementary faceted portion of the auxiliary tool 31. The anchoring elements 20 can thus be screwed into the bone or the vertebra 14-12 by turning the auxiliary tool 31 in the hollow, tubular guide member 30. The screw thread 20f at the same time engages the internal screw thread in the opening of the cramp element 24.

As already indicated above, the medical device according to the invention functions to orient bone elements or vertebrae relative to each other, for example if an intervertebral disc 16 or an intermediate vertebra 16' (see Figure 3B) is affected and has thus lost its functionality.

The two anchoring elements 20 of the medical device according to the invention are inserted into the vertebrae 12-14 by means of two identical guide member is 30-30', which vertebrae 12-14 are located on either side of the affected vertebra 16'. Since the two guide member is 30-30' are connected to the vertebrae 12-14 in question by means of the cramp elements 24, the two vertebrae 12-14 can be oriented relative to each other by suitably manipulating the two guide member is 30-30' so as to thus relieve the load on the affected vertebra 16' and prepare it for medical treatment.

In order to permanently relieve the load on the affected intermediate vertebra 16' during the medical treatment, the tool is provided with an adjusting mechanism 40, which is arranged for manipulating or orienting (in an x-y-z orientation) the two guide members 30-30' as well as the vertebrae 12-14 interconnected by means of the anchoring elements in question relative to each other.

Once the two vertebrae 12-14 have been oriented relative to each other by means of the guide members 30-30', their relative orientation can be fixated by means of the adjusting mechanism 14. Said adjusting mechanism 40 comprises two arms 42-42', which engage the two guide members 30-30', as well as an adjusting unit 41-43, for example in the form of an adjusting screw or spindle. Now that the two vertebrae 12-14 have been oriented relative to each other by means of the guide members 30-30' and the adjusting mechanism 40, the connecting element 18 built up of two element parts 18'-18" must be placed between the two anchoring elements 20.

To that end a so-called connecting ring 23 is first guided in the direction of the end 20d of the anchoring elements 20 that projects from the vertebrae 12-14 via the hollow, tubular guide member 30-30', using a suitable auxiliary tool 31. The connecting ring slips over the end 20d (and the faceted portion 20d') of the anchoring element 20. As shown in Figure 3C, the end 20d of the anchoring element 20 is also provided with an external screw thread 20e, which can mate with a corresponding internal screw thread of a nut 25, which can be screwed thereon and which locks the connecting ring 23 in position (see Figures 2, 3c and 4).

After the connecting ring 23 has been locked in position on the end 20d by means of the nut 24, the mutually different element parts 18'-18" of the connecting element 18 are inserted through the two hollow, tubular guide members 30-30' by means of a suitable auxiliary tool 31 (using the same auxiliary tool or different types of auxiliary tools 31).

As is clearly shown in Figure 2, the element part 18' is spatula-shaped, and the element part 18" is bar-shaped, which bar-shaped element part 18" can be fittingly received in the spatula shape of the other element part 18'. Also other embodiments of the construction in question are possible, however.

Since the guide member 30-30' guides the two element parts 18'-18" in the direction of the anchoring elements 20 in a vertical, i.e. a perpendicular, orientation relative to the patient's skin, a provision must be made to make it possible to reposition the two element parts 18'-18" to the orientation shown in Figure 2, whilst the underlying orientation of the vertebrae 12-14 by means of the guide members 30-30' and the adjusting mechanism remains ensured.

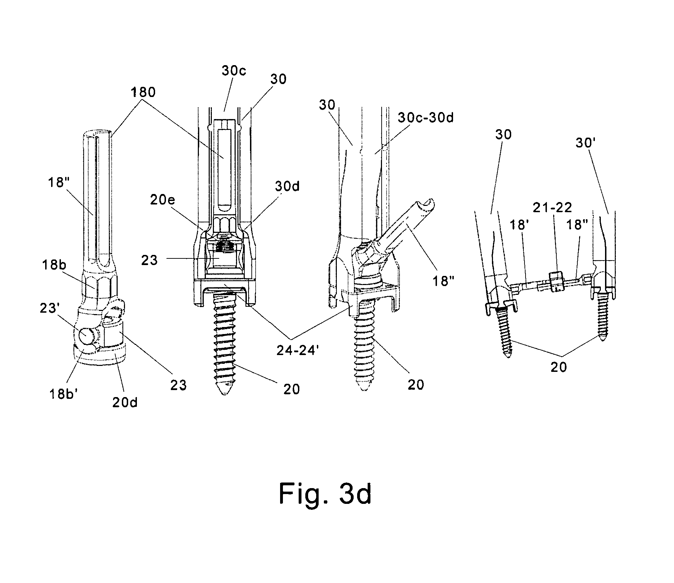

To that end, each guide member 30-30' is provided with a longitudinally extending opening 30d near its end 30a. The two element parts 18'-18" are provided with two hook-shaped members 18b' near their ends 18b, which can be hooked round cams 23' of the connecting rings 23. (See Figure 3D).

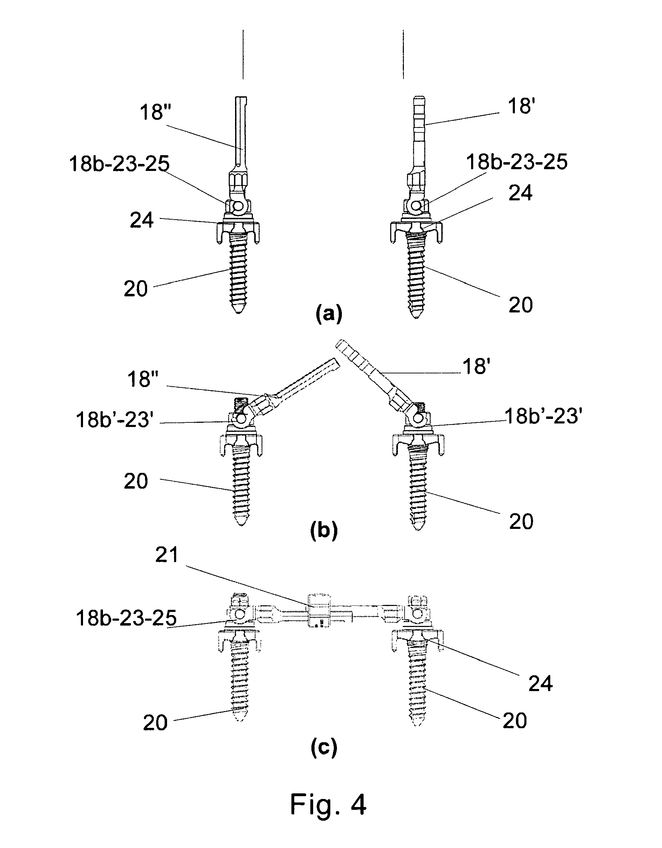

Once the two element parts 18'-18" have been connected, via the guide members 30-30', to the connecting ring 23 screwed onto the end 20d of the anchoring elements 20 (Figure 4A), the two element parts 18'-18" can be tilted (Figure 4B), which tilting movement is possible on account of the presence of the longitudinal opening 30d, which is present in the exterior surface of the guide members 30-30' near the end 30a thereof. Said tilting movement of the two element parts 18'-18" from the guide members is shown in Figure 3D and Figure 4B. The relative orientation of the vertebrae 12-14 during said tilting movement is guaranteed, since the adjusting mechanism 42 locks the two guide members 30-30' (which are still connected to the two vertebrae) in position.

In this way the element parts 18'-18" can be folded down, whilst maintaining the desired orientation of the two vertebrae 12-14, until the bar-shaped element part 18' is fittingly received in the spatula-shaped element part 18" (see Figure 2, Figure 3D and Figure 4C). The two element parts 18'-18" can subsequently be interconnected via the clamping element 21-22, thus permanently fixating and guaranteeing the relative orientation of the two vertebrae 12-14. The adjusting mechanism 42 can then be removed and the two guide members 30-30' can likewise be removed from the two vertebrae 12-14. The patient is thus only confronted with two minor surgical wounds, from which the two ends 20d of the anchoring elements 20 extend, thereby reducing the extent of the inconvenience to the patient and speeding up the patient's recovery.

It will be understood that the medical device and also the tool according to the invention provide a more versatile piece of equipment for the patient, which on the one hand makes it possible to realise a precise and reliable positioning of vertebrae relative to each other, but which on the other hand also significantly reduces the magnitude of the surgery, since less radical surgery is required, so that the extent of the inconvenience to the patient is significantly reduced and recovery is speeded up.

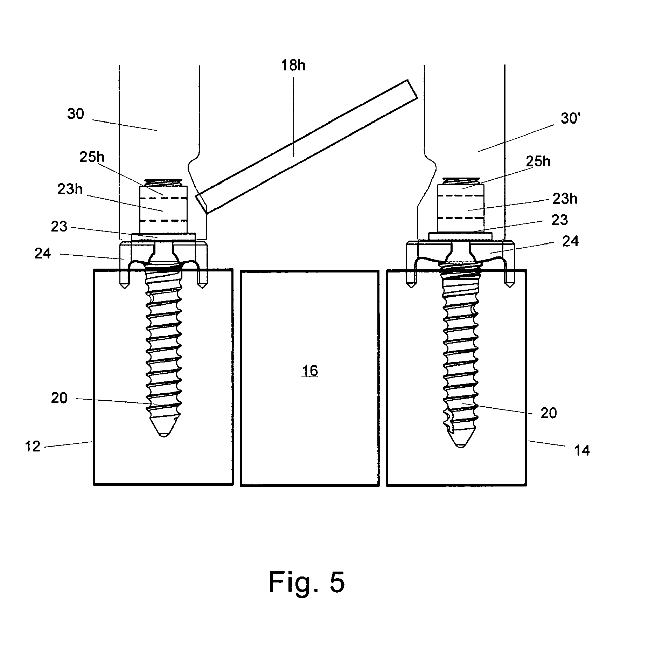

Figure 5 shows another embodiment of the medical device according to the invention. Parts corresponding to parts shown in the preceding Figures are indicated by the same numerals. In this embodiment, the connecting rings 23 are not provided with projecting cams which mate with hook-shaped parts of the element parts 18'-18" (see Figure 2). In this embodiment, the connecting element 18 is made in one piece and configured as a bar-shaped element 18h, which can be received in openings 23h in the connecting rings 23. The connecting element 18h can then be locked in position by means of bolts or screws 25h. Said locking bolts or nuts 25h are provided via the guide members 30-30' in this embodiment as well.

## Claims

1. A medical device for positioning bone elements, in particular vertebrae, relative to each other, comprising at least one assembly built up of two or more anchoring elements, which can each be implanted in a bone element with a first end thereof, as well as an elongated connecting element, which can be connected to the other ends of the anchoring elements projecting from the bone element in question, **characterised in that** a tool is provided for use in the placement, part by part, of the medical device, which tool comprises at least two hollow, tubular guide members, which guide members can each be positioned into abutment with a bone element for guiding the anchoring elements through the guide member and inserting said anchoring elements in the bone element, as well as an adjusting mechanism, which can be connected to the other ends of the guide members for positioning the guide members and the bone elements relative to each other.

2. A medical device according to claim 1, **characterised in that** the first end of each guide member is arranged for receiving a cramp-like element, which can be driven into the bone element.

3. A medical device according to claim 1 or 2, **characterised in that** the ends of the anchoring elements projecting from the bone element are each provided with a screw thread, which is to mate with a nut to be guided by the guide member, which nut functions to lock a connecting ring likewise to be guided by the guide member, which can be fitted over the end of the anchoring elements that projects from the bone element.

4. A medical device according to claim 3, **characterised in that** the elongated connecting element comprises two element parts to be guided by each guide member and to be connected to a connecting ring, as well as a connecting element for interconnecting said element parts.

5. A medical device according to claim 4, **characterised in that** the element parts and the connecting rings can be connected together by connecting means built up of hook-shaped members formed on the connecting rings or the element parts, which hook-shaped members hook round cams formed on the element parts or on the connecting rings.

6. A medical device according to claim 4 or 5, **characterised in that** one element part is bar-shaped and the other element part is spatula-shaped.

7. A medical device according to claim 4, 5 or 6, **characterised in that** each guide member is partially open in longitudinal direction near its first end for the purpose of releasing and interconnecting the element parts connected to the connecting ring in question.

8. A tool for use in the placement, part by part, of a medical device according to any one or more of the preceding claims.
